(19) Europäisches Patentamt
European Patent Office
Office européen des brevets

(11) Veröffentlichungsnummer: **0 470 489 A1**

# (12) EUROPÄISCHE PATENTANMELDUNG

(21) Anmeldenummer: **91112818.9**

(51) Int. Cl.[5]: **C07D 231/56**, A01N 43/56

(22) Anmeldetag: **30.07.91**

(30) Priorität: **04.08.90 DE 4024862**

(43) Veröffentlichungstag der Anmeldung:
**12.02.92 Patentblatt 92/07**

(84) Benannte Vertragsstaaten:
**AT BE CH DE DK ES FR GB GR IT LI NL**

(71) Anmelder: **HOECHST AKTIENGESELLSCHAFT**
**Postfach 80 03 20**
**W-6230 Frankfurt am Main 80(DE)**

(72) Erfinder: **Schütze, Rainer, Dr.**
**Am Flachsland 54**
**W-6233 Kelkheim/Ts.(DE)**
Erfinder: **Bauer, Klaus, Dr.**
**Doorner Strasse 53d**
**W-6450 Hanau(DE)**
Erfinder: **Bieringer, Hermann, Dr.**
**Eichenweg 26**
**W-6239 Eppstein/Ts.(DE)**

## (54) 4,5,6,7-Tetrahydro-3-aryl-indazole, Verfahren zu ihrer Herstellung und ihre Verwendung als Herbizide.

(57) Die Erfindung betrifft neue herbizide Verbindungen der Formel I oder I',

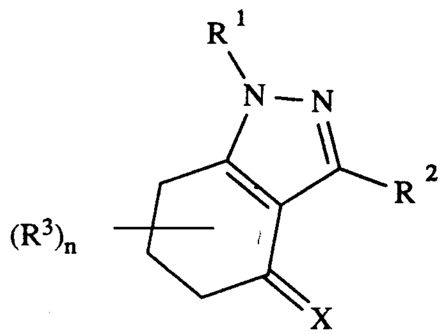

(I)

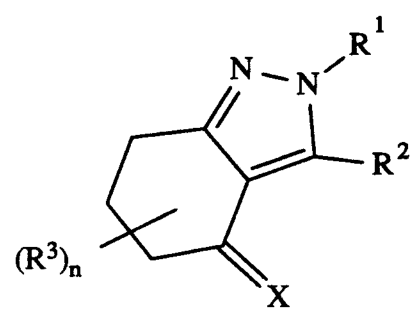

(I')

worin
- X Sauerstoff- oder Schwefelatom,
- $R^1$ H, $C_1$-$C_6$-Alkyl, $C_2$-$C_6$-Alkenyl, $C_2$-$C_6$-Alkinyl, $C_1$-$C_6$-Haloalkyl, ($C_1$-$C_4$-Alkoxy)-$C_1$-$C_4$-alkyl, ($C_1$-$C_6$-Alkoxy)-carbonyl, Aryloxycarbonyl, ($C_1$-$C_6$-Alkyl)-aminocarbonyl, Arylaminocarbonyl, Phenyl, substituiertes Phenyl oder Acyl,
- $R^2$ Phenyl oder substituiertes Phenyl,
- $R^3$ unabhängig von anderen Resten $R^3$ H oder $C_1$-$C_5$-Alkyl und
- n eine ganze Zahl von 0 bis 6

bedeuten.

Die Herbizide eignen sich zur selektiven Bekämpfung von Schadpflanzen in Getreidekulturen.

EP 0 470 489 A1

Die Erfindung betrifft das technische Gebiet der Planzenschutzmittel zur selektiven Bekämpfung von Unkräutern und Ungräsern in Nutzplanzenkulturen. Gegenstand der Erfindung sind 4,5,6,7-Tetrahydro-3-aryl-1H-indazole und -2H-indazole der Formel I oder I',

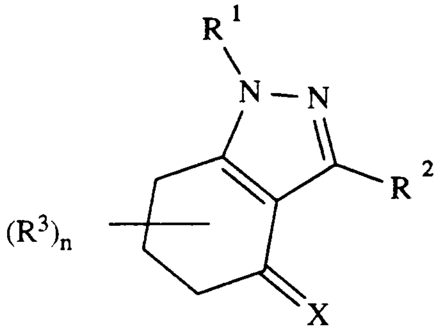

(I)

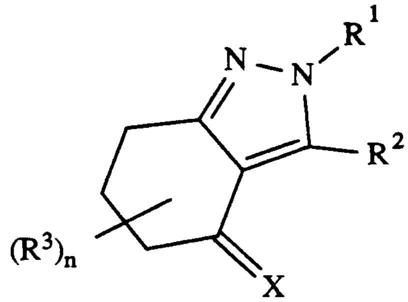

(I')

worin

X Sauerstoff- oder Schwefelatom,

$R^1$ H, $C_1$-$C_6$-Alkyl, $C_2$-$C_6$-Alkenyl, $C_2$-$C_6$-Alkinyl, $C_1$-$C_6$-Haloalkyl, ($C_1$-$C_4$-Alkoxy)-$C_1$-$C_4$-alkyl, ($C_1$-$C_6$-Alkoxy)-carbonyl, Aryloxycarbonyl, ($C_1$-$C_6$-Alkyl)-aminocarbonyl, Arylaminocarbonyl, Phenyl, substituiertes Phenyl oder Acyl,

$R^2$ Phenyl oder substituiertes Phenyl,

$R^3$ unabhängig von anderen Resten $R^3$ H oder $C_1$-$C_5$-Alkyl und

n eine ganze Zahl von 0 bis 6

bedeuten.

Hier und im folgenden können die Reste Alkyl, Alkenyl und Alkinyl als auch die den substituierten Alkylresten zugrundeliegenden Alkylteile sowohl geradkettig als auch verzweigt sein. Halogen bedeutet Fluor, Chlor, Brom oder Iod, vorzugsweise Fluor oder Chlor. Aryl bedeutet ein carbocyclisches oder heterocyclisches, gegebenenfalls substituiertes aromatisches Ringsystem, vorzugsweise gegebenenfalls substituiertes Phenyl. Substituiertes Phenyl bedeutet vorzugsweise Phenyl, das durch 1 bis 5 Reste, insbesondere 1 bis 3 Reste aus der Gruppe Halogen, Alkyl, Alkoxy, Haloalkyl, Haloalkoxy, Cyano, Nitro, Alkanoyl, Alkanoylamino, Amino. Mono- und Dialkylamino, Carboxy und Derivate der Carboxygruppe, wie Alkoxycarbonyl, Mono- und Dialkylaminocarbonyl substituiert ist. Dabei sind bei den Resten mit Kohlenstoffketten solche bevorzugt, die 1 bis 6, insbesondere 1 bis 4 C-Atome enthalten. Haloalkyl oder Haloalkoxy bedeutet Alkyl bzw. Alkoxy, die mit einem oder mehreren Atomen aus der Gruppe Halogen substituiert sind. Acyl bedeutet eine Carbonylgruppe, die an einen aliphatischen, araliphatischen oder einen Aryl-rest gebunden ist, vorzugsweise Alkylcarbonyl.

Die erfindungsgemäßen Verbindungen umfassen Derivate vom Typ des Tetrahydro-1H-indazols und Tetrahydro-2H-indazols, die regioisomere Verbindungen darstellen. Bei den Verfahren zur deren Herstellung entstehen die regioisomeren Verbindungen der Formeln I und I' häufig gemeinsam. Die Verbindungen der Formel I' sind ebenfalls herbizid wirksam, jedoch sind die Verbindungen der Formel I bevorzugt.. Wenn nicht näher spezifiziert, sind im folgenden mit "erfindungsgemäßen Verbindungen" Verbindungen der Formel I oder der Formel I' oder Gemische aus Verbindungen der Formeln I und I' gemeint.

Manche Verbindungen der Formel I und I' enthalten ein oder mehrere asymmetrische C-Atome oder auch Doppelbindungen, die in den allgemeinen Formeln I bzw I' nicht gesondert angegeben sind. Die durch ihre spezifische Raumform definierten möglichen Stereoisomeren, wie Enantiomere, Diastereomere, Z- und E-Isomere sind jedoch alle von den Formeln I bzw. I' umfaßt und können nach üblichen Methoden aus Gemischen der Stereoisomeren erhalten werden oder auch durch stereoselektive Reaktionen in Kombination mit dem Einsatz von stereochemisch reinen Ausgangsstoffen hergestellt werden. Die genannten Stereoisomeren in reiner Form als auch ihre Gemische sind somit Gegenstand dieser Erfindung.

Von besonderem Interesse sind erfindungsgemäße Verbindungen der Formeln I oder I', worin

$R^1$ H, $C_1$-$C_4$-Alkyl, $C_2$-$C_4$-Alkenyl, $C_2$-$C_4$-Alkinyl, $C_1$-$C_4$-Haloalkyl, ($C_1$-$C_2$-Alkoxy)-$C_1$-$C_4$-alkyl, ($C_1$-$C_4$-Alkoxy)-carbonyl, ($C_1$-$C_4$-Alkyl)-aminocarbonyl, Phenyloxycarbonyl, Phenylaminocarbonyl, Phenyl,

wobei die 3 letztgenannten Gruppen jeweils im Phenylring unsubstituiert oder durch ein oder mehrere Reste aus der Grupppe Halogen, $C_1$-$C_4$-Alkyl, $C_1$-$C_4$-Haloalkyl, $C_1$-$C_4$-Alkoxy, $C_1$-$C_4$-Haloalkoxy, Nitro, Carboxy und Säurederivate von Carboxy, insbesondere $C_1$-$C_4$-Alkylester, $C_2$-$C_4$-Alkenylester, $C_2$-$C_4$-Alkinylester, Amid, $C_1$-$C_4$-Alkylamid und Di-($C_1$-$C_4$-Alkyl)amid substituiert sind, oder Acyl, insbesondere $C_1$-$C_6$-Alkanoyl, bedeutet.

$R^1$ ist vorzugsweise H, Methyl, Ethyl, n- oder i-Propyl, n-, i-, t- oder 2-Butyl, Allyl, Propargyl, Trifluormethyl, 2,2,2-Trifluorethyl, Methoxymethyl, Ethoxymethyl, 1- und 2-Methoxyethyl, 1- und 2-Ethoxyethyl, Phenyloxycarbonyl, Phenylaminocarbonyl, Phenyl, wobei die 3 letztgenannten Gruppen jeweils im Phenylring unsubstituiert oder durch 1 bis 3 Reste aus der Gruppe Halogen, $C_1$-$C_4$-Alkyl, Trifluormethyl, $C_1$-$C_4$-Alkoxy, Nitro, ($C_1$-$C_4$-Alkoxy)-carbonyl, Mono- und Di-($C_1$-$C_4$-Alkylamino)-carbonyl und $C_2$-$C_3$-Alkanoyl substituiert sind, Formyl oder Acetyl.

Von besonderem Interesse sind auch erfindungsgemäße Verbindungen der Formel I oder I', worin

$R^2$ Phenyl, das unsubstituiert oder durch ein bis drei Reste aus der Gruppe Halogen, Nitro, $C_1$-$C_4$-Alkyl, $C_1$-$C_4$-Halogenalkyl und $C_1$-$C_4$-Alkylsulfonyl substituiert ist, bedeutet.

$R^2$ ist vorzugsweise Phenyl, das durch zwei nicht gleiche Reste aus der Gruppe Chlor, Nitro, Methansulfonyl und Trifluormethyl substituiert ist.

Die Reste $R^3$ sind unabhängig voneinander vorzugsweise H oder Methyl; n ist vorzugsweise 0 bis 4, insbesondere 0 bis 3;

X ist vorzugsweise ein Sauerstoffatom.

Gegenstand der Erfindung ist auch ein Verfahren zur Herstellung der erfindungsgemäßen Verbindungen der Formeln I oder I', dadurch gekennzeichnet, daß man

a) Verbindungen der Formel II,

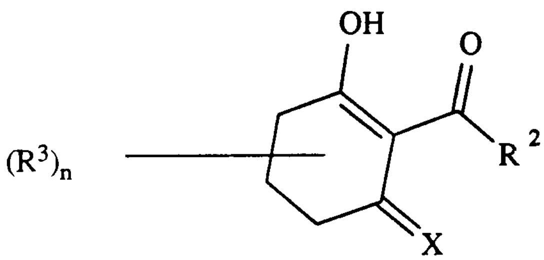

(II)

worin $R^2$ und $R^3$ und X wie bei der obengenannten Formel I bzw. I' definiert sind, mit Hydrazinderivaten der Formel III,

$R^1 - NH - NH_2$ (III)

worin $R^1$ wie bei der obengenannten Formel I bzw. I' definiert ist, umsetzt oder

b) für den Fall, daß $R^1$ = Alkyl ist, Verbindungen der genannten Formel I bzw I', in denen $R^1$ ein Wasserstoffatom bedeutet, mit geeigneten Alkylierungsmitteln umsetzt oder

c) ausgenommen zur Herstellung von Verbindungen der Formel I', Cyclohexan-1,3-dione der Formel IV mit Chlorhydrazonen der Formel V,

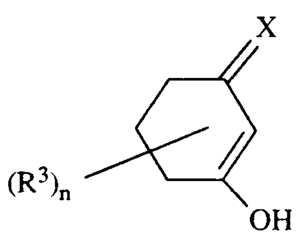

(IV)

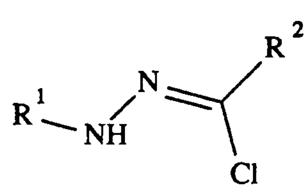

(V)

wobei $R^1$ bis $R^3$ und X wie bei der genannten Formel I bzw. I' definiert sind, in Gegenwart einer Base zu Verbindungen der genannten Formel I umsetzt.

Die Umsetzung der Verbindung der Formel II nach Variante a) wird vorzugsweise in polaren, protischen

(organischen) Lösungsmitteln, insbesondere Alkoholen wie z.B. Methanol, Ethanol, Propanol, n-Butanol, Isobutanol, tert.-Butanol und 2-Butanol bei Temperaturen von Raumtemperatur bis zum Siedepunkt des Reaktionsgemisches, insbesondere bei Rückflußtemperatur durchgeführt.

Die für die Umsetzung des an Position 1 unsubstituierten Indazols der Formel I bzw. I' geeigneten Alkylierungsmittel nach Varante b) (vgl. folgendes Schema)

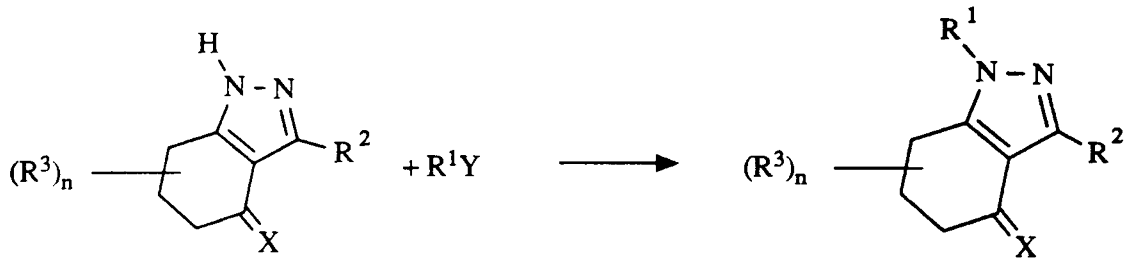

sind beispielsweise übliche Alkylierungsmittel $R^1Y$(Y = Abgangsgruppe) wie Dialkylsulfate, Alkylhalogenide, Methan- und p-Toluolsulfonsäurealkylester und werden in üblicher Weise eingesetzt. Beispielsweise kann eine Methylierung mit Dimethylsulfat in alkalischer Lösung durchgeführt werden, vorzugsweise in Natronlauge oder alkalischer Alkohollösung.

Der Aufbau des bicyclischen Ringsystems bei der Umsetzung nach Variante c) erfolgt als 1,3-dipolare Cycloaddition und kann beispielsweise sowohl in aprotischen (organischen) Lösungsmitteln wie Toluol oder Dioxan als auch in polaren, protischen (organischen) Lösungsmitteln, wie z.B. den Alkoholen Methanol und Ethanol durchgeführt werden.

Die für die Herstellung der erfindungsgemäßen Verbindungen nach Variante a) benötigten Verbindungen der Formel II lassen sich nach oder analog literaturbekannten Verfahren synthetisieren; siehe z. B. J. Chem. Soc. **1953** 803, J. Chem. Soc. **1955** 341, Tetrahedron Lett. **29**, 2491 (1975), J. prakt. Chemie **141**, 149 (1934),DE-A-3902818, EP-A-249150, US-A-4780127.

Die Hydrazinderivate III lassen sich ebenfalls nach oder analog literaturbekannten Methoden herstellen; siehe z. B. J. Am. Chem. Soc. **76**, 4869 (1954), Chem. Ber. **81**, 81 (1948), J. Chem. Soc. **1958** 4723, Org. Synth. **35**, 28 (1955), J. Am. Chem. Soc. **80**, 5786 (1958), J. Org. Chem. **46**, 5413 (1981)).

Die Cyclohexan-1,3-dione der Formel IV sind teilweise im Handel erhältlich oder können nach bekannten Verfahren hergestellt werden; siehe z. B. J. Chem. Soc. **1953**, 811 und Chem. Pharm. Bull. **31**, 1518 (1983).

Die Chlorhydrazone der Formel V, die ebenfalls als Ausgangsstoffe für die Synthese der 1,3-dipolaren Nitrilimine in Variante c) dienen, können nach bekannten Verfahren hergestellt werden; siehe z.B Houben-Weyl Bd. 2, S.448 und Tetrahedron **17**, 3 (1962)).

Bei der Umsetzung gemäß Varianten a) und b) können insbesondere regioisomere Verbindungen entstehen.

Die erfindungsgemäßen Verbindungen der Formel I bzw I' weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den perennierenden Unkräutern.

Unter den spezifischen Kulturbedingungen im Reis vorkommende Unkräuter wie z.B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräter wachsen bis

zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der erfindungsgemäßen Verbindungen der Formel I beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate (SL), emulgierbare Konzentrate (EC), Emulsionen (EW) wie Öl-in-Wasser- und Wasser-in-Öl-Emulsionen, versprühbare Lösungen oder Emulsionen, Suspensionskonzentrate(SC), Dispersionen auf Öl- oder Wasserbasis, ölmischbare Lösungen (OL) Suspoemulsionen, Kapselsuspensionen (CS), Stäubemittel (DP), Beizmittel, Granulate zur Boden- oder Streuapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, G. Hauser Verlag München, 4. Aufl. 1986; van Valkenburg, "Pesticides Formulations", Marcel Dekker N.Y. 1973; K Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Galdwell N.J.; H.v. Olphen, "Introduction to Clay Colloid Ghemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MG Publ. Corp., Ridgewood N.J.; Sisley and Wood, "Encyclopedia of Surface Active Agents", Chem. Publ. Co. Inc., N.Y 1964; Schönfeldt, "Grenzflächenaktive Äthylenoxidaddukte", Wiss. Verlagsgesell., Stuttgart 1976; Winnacker-Küchler, "Chemische Technologie", Band 7, G. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxyethylierte Alkylphenole, polyoxethylierte Fettalkohole und Fettamine, Fettalkoholpolyglykolethersulfate, Alkansulfonate oder Alkylarylsulfonate, und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyglycolether, Sorbitanfettäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophillit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Teller-, Fließbett-, Extruder- und Sprühgranulate können nach üblichen Verfahren hergestellt werden;

siehe z.B. Verfahren in "Spray Dyring Handbook", 3rd Ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, s. 8-57.

Für weitere Informationen zur Formulierung von Pflanzenschutzmitteln siehe z.B. G.G. Klingman, "Weed Control as a Science", John Wiley and Sons, Inc., New York, 1961, Seiten 81-96 und J.D. Freyer' s. A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gewichtsprozent, insbesondere 0,1 bis 95 Gew.-%, Herbizid-Wirkstoff.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80 Gew.-% betragen. Staubförmige Formulierungen enthalten meistens 1 bis 20 Gew.-% an Wirkstoff, versprühbare Lösungen etwa 0,2 bis 20 Gew.-%. Bei Granulaten hängt der Wirkstoffgehalt zum Teil davon ab, ob die wirksame Verbindung flüssig oder fest vorliegt. Meist liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der erfindungsgemäßen Verbindungen. Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Auch Mischungen oder Mischformulierungen mit anderen Wirkstoffen, wie z.B. Insektiziden, Akariziden, Herbiziden, Safenern, Düngemitteln, Wachstumsregulatoren oder Fungiziden sind gegebenenfalls möglich.

Folgende Beispiele dienen zur Erläuterung der Erfindung:

A) **Formulierungsbeispiele**

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inerstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether ((R)Triton X 207), 3 Gew.-Teilen Isotridecanolpolyglykolether (8 EO) und 71 Gew.-Teilen paraffinischem Mineralöl (Siedebereich z.B. ca 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertem Nonylphenol als Emulgator.

e) Ein in Wasser dispergierbares Granulat wird erhalten, indem man

| | | |
|---|---|---|
| 75 Gewichtsteile einer Verbindung der Formel (I), | | |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

25 Gewichtsteile einer Verbindung der Formel (I),

| | | |
|---|---|---|
| 5 | " | 2,2'-dinaphthylmethan-6,6'-disulfonsaures Natrium, |
| 2 | " | oleolymethyltaurinsaures Natrium, |
| 1 | " | Polyvinylalkohol, |
| 17 | " | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

g) Ein Extruder-Granulat erhält man, indem man 20 Gewichtsteile Wirkstoff der Formel (I), 3 Gewichtsteile ligninsulfonsaures Natrium, 1 Gewichtsteil Carboxymethylcellulose und 76 Gewichtsteile Kaolin vermischt, vermahlt und mit Wasser anfeuchtet. Dieses Gemisch wird extrudiert und anschließend im Luftstrom getrocknet.

h) Analog zu den vorstehenden Beispielen a) bis g) werden Formulierungen mit Verbindungen der Formel I' erhalten, wenn man letztere anstelle von Verbindungen der Formel I einsetzt.

B) **Herstellungsbeispiele**

**4,5,6,7-Tetrahydro-4-oxo-3-(2-nitrophenyl)-1H-indazol (zu Beispiel 1, Tabelle 1)**

3,2 g (12,2 mmol) 2-Nitrobenzoylcyclohexan-1,3-dion werden mit 0,52 g (12,2 mmol) Hydrazinhydrat (80%) in 120 ml Ethanol 4h im Rückfluß erwärmt. Anschließend destilliert man das Ethanol unter reduziertem Druck ab und fügt 80 ml Methanol hinzu. Der ausgefallene Feststoff wird abgesaugt und im Vakuum getrocknet. Man erhält 1,41 g (45 % d.Th.) 4,5,6,7-Tetrahydro-4-oxo-3-(2-nitrophenyl)-1H-indazol der Formel

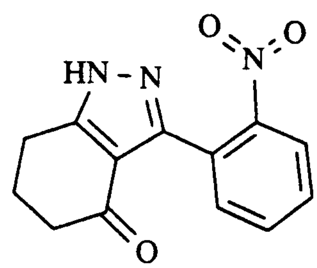

mit einem Schmelzpunkt von 236° C (Zers.).

**4,5,6,7-Tetrahydro-4-oxo-3-(4-chlor-2-nitrophenyl)-1-ethyl-1H-indazol (zu Beispiel 2, Tabelle 1)**

2,08 g (7,13 mmol) 4,5,6,7-Tetrahydro-4-oxo-3-(4-chlor-2-nitrophenyl)-1H-indazol werden in 80 ml 10% wäßriger Natronlauge gelöst, filtriert vom Ungelösten ab und fügt der Lösung dann 1,2 g (7,8 mmol) Diethylsulfat hinzu. Anschließend wird 1,5 h bei Raumtemperatur und 1 h bei 0° C nachgerührt, wobei nach ca. 10 min bereits ein Niederschlag auszufallen beginnt. Der Niederschlag wird nach Beendigung der Reaktion abgesaugt und an Kieselgel mit Essigsäureethylester/Dichlormethan (1:2) chromatografiert. Die Fraktion mit $R_f = 0.72$ enthält 0,34 g (15,3% d.Th.) gewünschtes Produkt der Formel

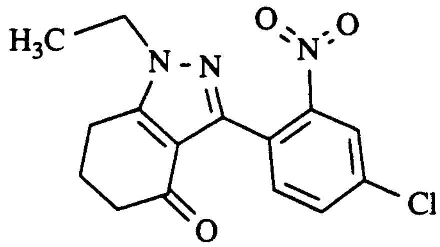

mit einem Schmelzpunkt von 184 ° C.

**4,5,6,7-Tetrahydro-4-oxo-1,3-diphenyl-1H-indazol (zu Beispiel 4, Tabelle 1)**

3 g Benz-phenylhydrazidchlorid werden zusammen mit 5,84g Cyclohexan-1,3-dion in 80 ml Toluol gelöst und 1,4g Triethylamin hinzugefügt. Die Lösung wird 4h auf 80-90 ° C erhitzt. Anschließend läßt man auf Raumtemperatur abkühlen und wäscht mit 1 n HCl und dann mit Natriumhydrogencarbonatlösung. Die organische Phase wird getrocknet und das Toluol unter reduziertem Druck abgezogen. Man nimmt den Rückstand in Methanol auf und läßt langsam kristallisieren. So erhält man 1,3 g (35 % d.Th.) gewünschtes Produkt der Formel

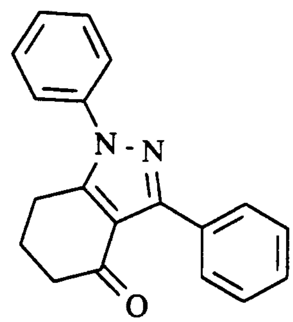

mit einem Schmelzpunkt von 162 ° C.

**4,5,6,7-Tetrahydro-4-oxo-1-methyl-3-(2-chlor-4-methansulfonyl-phenyl)-1H-indazol (zu Beispiel 6, Tabelle 1)**

2 g 2-(2-Chlor-4-methansulfonylbenzoyl)cyclohexan-1,3-dion werden in 50 ml Ethanol gelöst, 0,28 g Methylhydrazin hinzugefügt und die Lösung dann 4 h unter Rückfluß erhitzt. Anschließend läßt man abkühlen und saugt den ausgefallen Niederschlag ab. Man erhält 0,85 g (41 % d.Th.) des gewünschten Produkts der Formel

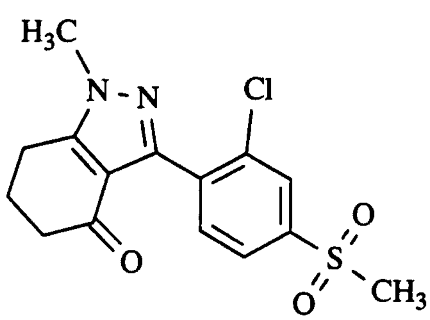

mit einem Schmelzpunkt von 208 °C.

In der folgenden Tabelle 1 sind die Beispiele 1,2,4 und 6 zusammen mit weiteren Beispielen der allgemeinen Formel VI aufgeführt, die in analoger Weise erhalten werden:

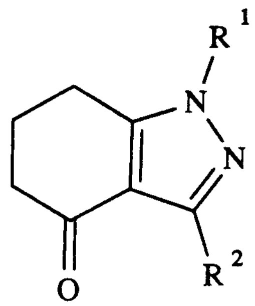

(VI)

**Tabelle 1**

| Beispiel | $R^2$ | $R^1$ | Schmp.[$^0C$] |
|---|---|---|---|
| 1 | 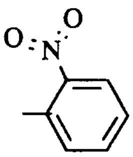 | H | 236 |
| 2 | 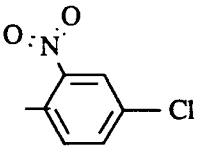 | $C_2H_5$ | 184 |
| 3 | 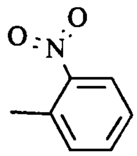 | 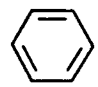 | 221 |
| 4 | 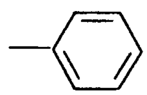 | 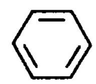 | 162 |
| 5 | 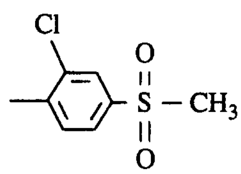 | 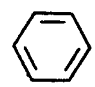 | 115 - 118 |
| 6 | 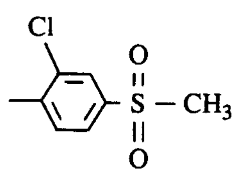 | $CH_3$ | 208 |
| 7 | 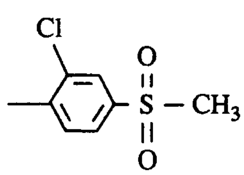 | H | 222 |
| 8 | 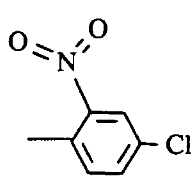 | H | 242 |

| Beispiel | $R^2$ | $R^1$ | Schmp.[$^0C$] |
|---|---|---|---|
| 9 | 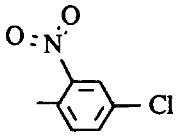 | 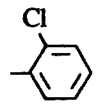 | 138 - 141 |
| 10 | 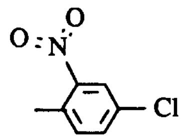 | 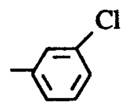 | 128 |
| 11 | 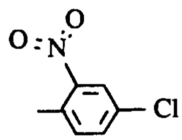 | 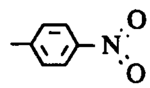 | 100 - 103 |
| 12 | 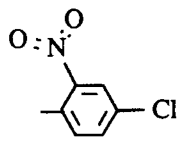 | 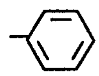 | 85 |
| 13 | 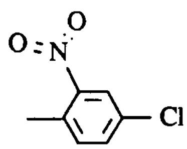 | $CH_3$ | 215 |
| 14 | 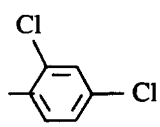 | 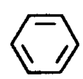 | 154 |
| 15 | 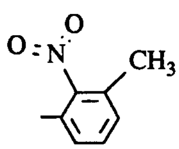 | 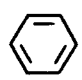 | 193 |
| 16 | 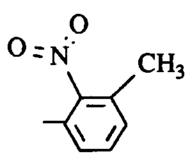 | H | 213 |

| Beispiel | $R^2$ | $R^1$ | Schmp.[$^0$C] |
|---|---|---|---|
| 17 | 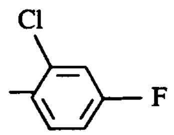 | H | 191 |
| 18 | 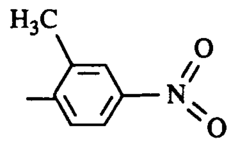 | $CH_3$ | 213 |
| 19 | 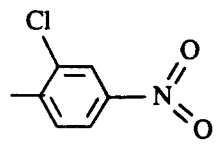 | $CH_3$-C=O | 131 |
| 20 | 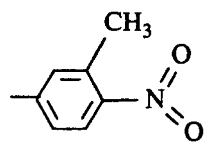 | $CH_3$ | 175 |
| 21 | 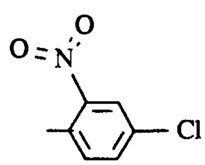 | $CH_2$-$CF_3$ | 182 |
| 22 | 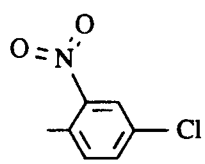 | t.$C_4H_9$ | 250 |
| 23 | 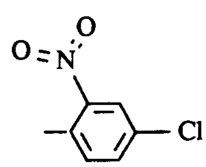 | n.$C_3H_7$ | 144 - 148 (Isomerengemisch) |
| 24 | 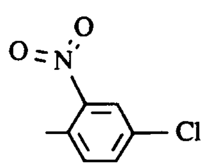 | n.-$C_4H_9$ | |

| Beispiel | $R^2$ | $R^1$ | Schmp.[$^0C$] |
|---|---|---|---|
| 25 | 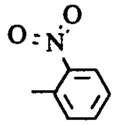 | $CH_3$ | |
| 26 | 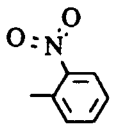 | $C_2H_5$ | |
| 27 | 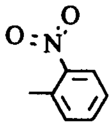 | $CH_2CF_3$ | |
| 28 | 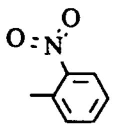 | $t.C_4H_9$ | |
| 29 | 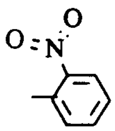 | $n.C_3H_7$ | |
| 30 | 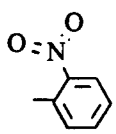 | $n.C_4H_9$ | |
| 31 | 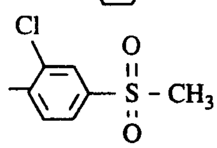 | $CH_2CF_3$ | 95 (Isomerengemisch) |
| 32 | 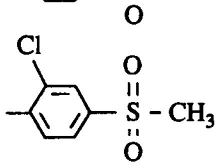 | $C_2H_5$ | |
| 33 | 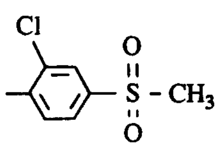 | $t.C_4H_9$ | |

| Beispiel | $R^2$ | $R^1$ | Schmp.[$^0C$] |
|---|---|---|---|
| 34 | 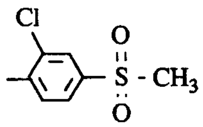 | $n.C_3H_7$ | |
| 35 | 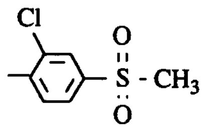 | $n.C_4H_9$ | |
| 36 | 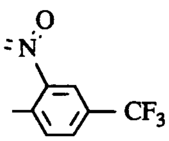 | 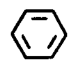 | 79 |
| 37 | 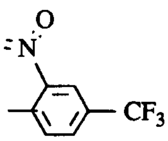 | $CH_3$ | 168 |
| 38 | 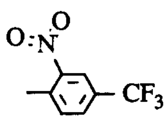 | H | 214 |
| 39 | 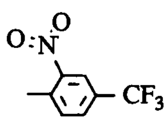 | $CH_2CF_3$ | |
| 40 | 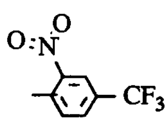 | $C_2H_5$ | |
| 41 | 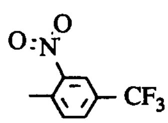 | $t.C_4H_9$ | |
| 42 | 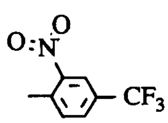 | $C_3H_7$ | |

| Beispiel | $R^2$ | $R^1$ | Schmp.[$^0$C] |
|---|---|---|---|
| 43 | 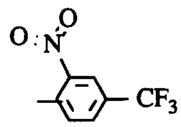 | $n.C_4H_9$ | |
| 44 | 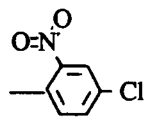 | $-CO-CH_3$ | 222 |
| 45 | " | $-CO-OC_2H_5$ | 138 |
| 46 | " | $-CO-NH-C_2H_5$ | 192 |
| 47 | " | $-CO-NH-i-C_3H_7$ | 188 |
| 48 | " | 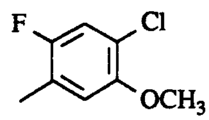 | 92-95 |
| 49 | " | -CO-H | 183 |
| 50 | " | $-CO-O-C_6H_5$ | 261 |
| 51 | " | 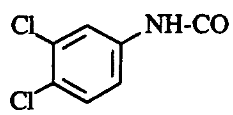 | 216-218 |
| 52 | $-C_6H_5$ | $-CH_3$ | 105 |
| 53a | 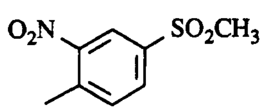 | $-CH_3$ | |

| Beispiel | $R^2$ | $R^1$ | Schmp.[$^0C$] |
|---|---|---|---|
| 53b | 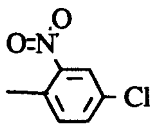 | $-CO-NH-t-C_4H_9$ | 175 |
| 53c | 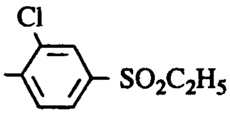 | H | 226 |
| 53d | " | $CH_3$ | 75 - 81 (Isomerengemisch) |
| 53e | 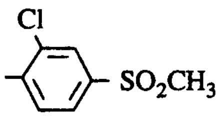 | - 4 -Chlorphenyl | 168 - 175 |

In analoger Weise wie die Verbindungen aus der vorstehenden Tabelle 1 werden die in der folgenden Tabelle 2 aufgeführten Verbindungen der allgemeinen Formel VII erhalten:

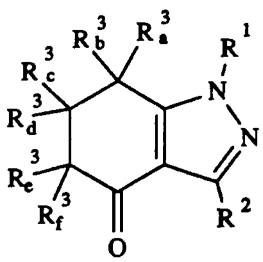

(VII)

**Tabelle 2**

| Beispiel | $R^2$ | $R^1$ | $R_a^3$ | $R_b^3$ | $R_c^3$ | $R_d^3$ | $R_e^3$ | $R_f^3$ | Schmp.[$^0$C] |
|---|---|---|---|---|---|---|---|---|---|
| 54 | 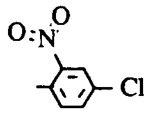 | $CH_3$ | H | H | H | H | $CH_3$ | $CH_3$ | 132 |
| 55 | 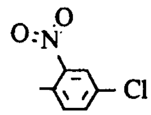 | $CH_3$ | $CH_3$ | H | H | H | $CH_3$ | $CH_3$ | |
| 56 | 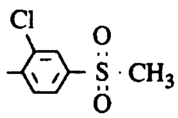 | $CH_3$ | H | H | H | H | $CH_3$ | $CH_3$ | 170 - 175 (Isom.gemisch) |
| 57 | 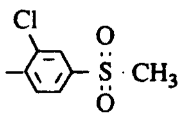 | $CH_3$ | $CH_3$ | H | H | H | $CH_3$ | $CH_3$ | 182 - 190 |

| Beispiel | $R^2$ | $R^1$ | $R_a^3$ | $R_b^3$ | $R_c^3$ | $R_d^3$ | $R_e^3$ | $R_f^3$ | Schmp.[$^0$C] |
|---|---|---|---|---|---|---|---|---|---|
| 58 | 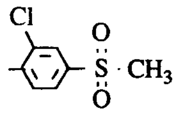 | H | H | H | $CH_3$ | $CH_3$ | H | H | |
| 59 | 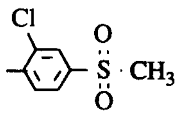 | $CH_3$ | H | H | $CH_3$ | $CH_3$ | H | H | |
| 60 | 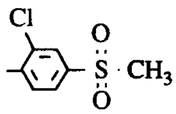 | Ph | H | H | $CH_3$ | $CH_3$ | H | H | |
| 61 | 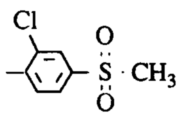 | $t.C_4H_9$ | H | H | $CH_3$ | $CH_3$ | H | H | |
| 62 | 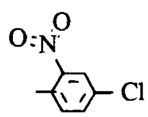 | H | H | H | H | H | $CH_3$ | $CH_3$ | |
| 63 | 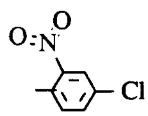 | H | $CH_3$ | H | H | H | $CH_3$ | $CH_3$ | |
| 64 | 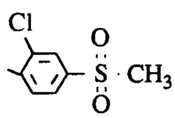 | H | H | H | H | H | $CH_3$ | $CH_3$ | |
| 65 | 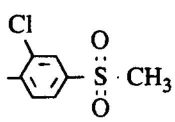 | H | $CH_3$ | H | H | H | $CH_3$ | $CH_3$ | |
| 66 | 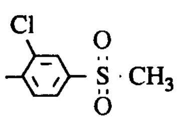 | Ph | H | H | H | H | $CH_3$ | $CH_3$ | |

| Beispiel | $R^2$ | $R^1$ | $R_a^3$ | $R_b^3$ | $R_c^3$ | $R_d^3$ | $R_e^3$ | $R_f^3$ | Schmp.[$^0$C] |
|---|---|---|---|---|---|---|---|---|---|
| 67 | 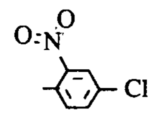 | i.$C_3H_7$ | H | H | $CH_3$ | $CH_3$ | H | H | |
| 68 | 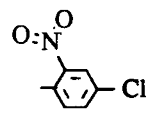 | $C_2H_5$ | H | H | $CH_3$ | $CH_3$ | H | H | |
| 69 | 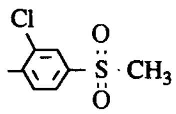 | $C_2H_5$ | H | H | $CH_3$ | $CH_3$ | H | H | |
| 70 | 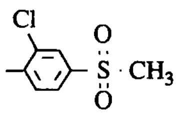 | n.$C_3H_7$ | H | H | $CH_3$ | $CH_3$ | H | H | |
| 71 | 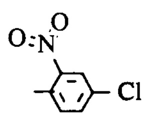 | H | H | H | $CH_3$ | $CH_3$ | H | H | 231 |
| 72 | 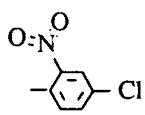 | $CH_3$ | H | H | $CH_3$ | $CH_3$ | H | H | 158 - 165 |
| 73 | 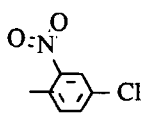 | Ph | H | H | $CH_3$ | $CH_3$ | H | H | |
| 74 | 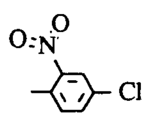 | t.$C_4H_9$ | H | H | $CH_3$ | $CH_3$ | H | H | |
| 75 | 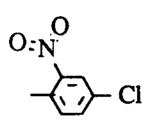 | n.$C_3H_7$ | H | H | $CH_3$ | $CH_3$ | H | H | |

| Beispiel | $R^2$ | $R^1$ | $R_a^3$ | $R_b^3$ | $R_c^3$ | $R_d^3$ | $R_e^3$ | $R_f^3$ | Schmp.[°C] |
|---|---|---|---|---|---|---|---|---|---|
| 76 | 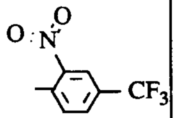 | H | H | H | $CH_3$ | $CH_3$ | H | H | |
| 77 | 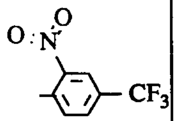 | $CH_3$ | H | H | $CH_3$ | $CH_3$ | H | H | |
| 78 | 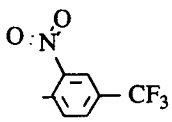 | Ph | H | H | $CH_3$ | $CH_3$ | H | H | |
| 79 | 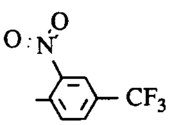 | $C_2H_5$ | H | H | $CH_3$ | $CH_3$ | H | H | |
| 80 | 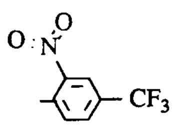 | $t.C_4H_9$ | H | H | $CH_3$ | $CH_3$ | H | H | |
| 81 | 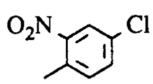 | $C_2H_5OCO$ | H | H | $CH_3$ | $CH_3$ | H | H | 116 |
| 82 | " | $CH_3CO$ | H | H | $CH_3$ | $CH_3$ | H | H | 170 |
| 83 | " | $C_6H_5OCO$ | H | H | $CH_3$ | $CH_3$ | H | H | 176 |
| 84 | " | $C_2H_5NHCO$ | H | H | $CH_3$ | $CH_3$ | H | H | 200 |
| 85 | " | $i\text{-}C_3H_7NHCO$ | H | H | $CH_3$ | $CH_3$ | H | H | 204 |
| 86 | " | H-CO | H | H | $CH_3$ | $CH_3$ | H | H | 162 |
| 87 | " | $CH_2CF_3$ | H | H | $CH_3$ | $CH_3$ | H | H | 115 |

C) **Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0-5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

1. Unkrautwirkung im Vorauflauf

Samen bzw. Rhizomstücke von mono- und dikoytylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600-800 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3-4 Wochen im Vergleich zu unbehandelten Kontrollen.
Wie die Boniturwerte in Tabelle 1 zeigen, weisen die erfindungsgemäßen Verbindungen eine gute herbizide Vorauflaufwirksamkeit gegen ein breites Spektrum von Ungräsern und Unkräutern auf.

Tabelle 3: Vorauflaufwirkung

| Beispiel | Dosis (kg a.i./ha) | herbizide Wirkung SIAL | CRSE | STME | ECCR |
|---|---|---|---|---|---|
| 5 | 1,25 | 3 | 5 | 5 | 5 |
| 1 | 1,25 | 3 | 4 | 5 | 5 |
| 8 | 1,25 | 5 | 4 | 5 | 3 |
| 13 | 1,25 | 5 | 5 | 5 | 5 |
| 15 | 1,25 | ohne Wirkung | | | |

Abkürzungen:
SIAL = Sinapis alba
CRSE = Chrysanthemum segetum
STME = Stellaria media
ECCR = Echinochloa crus-galli
a.i. = Aktivsubstanz

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.
Die als Spritzpulver bzw. als Emulsionskonzentrate formulierten erfindungsgemäßen Verbindungen wurden in verschiedenen Dosierungen mit einer Wasseraufwandmenge von umgerechnet 600-800 l/ha auf die grünen Pflanzenteile gesprüht und nach ca. 3-4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 4).

Tabelle 4: Nachauflaufwirkung

| Beispiel | Dosis (kg a.i./ha) | herbizide Wirkung SIAL | CRSE | STME | ECCR |
|---|---|---|---|---|---|
| 5 | 1,25 | 4 | 2 | 5 | 1 |
| 1 | 1,25 | 3 | 1 | 2 | 1 |
| 8 | 1,25 | 4 | 2 | 4 | 3 |
| 13 | 1,25 | 5 | 1 | 5 | 3 |
| 15 | 1,25 | 4 | 0 | 4 | 1 |

Abkürzungen:
SIAL = Sinapis alba
CRSE = Chrysanthemum segetum
STME = Stellaria media
ECCR = Echinochloa crus-galli
a.i. = Aktivsubstanz

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter 1. beschrieben behandelt, die übrigen im Gewächshaus aufgestellt, bis die Pflanzen zwei bis drei echte Blätter entwickelt hatten, und dann mit den erfindungsgemäßen Substanzen in unterschiedlichen Dosierungen wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kortoffeln im Vor- und Nachauflaufverfahren selbst bei hohen Wirkstoffdosierungen ungeschädigt ließen. Einige Substanzen schonten darüber hinaus auch Gramineen-Kulturen wie zu.B. Gerste, Weizen, Roggen, Sorghum-Hirsen, Mais oder Reis. Die erfindungsgemäßen Verbindungen weisen somit eine hohe Selektivität bei Anwendung zur Bekämpfung von unerwünschtem Pflanzenwuchs in landwirtschaftlichen Kulturen auf.

4. Herbizide Wirkung bei Anwendung in Reis

Knollen und Rhizome bzw. Jungpflanzen oder Samen verschiedener Reisunkräuter wie Cyperus-Arten, Eleocharis, Scirpus und Echinochloa wurden in geschlossenen Plastiktöpfen in spezielle Reiserde ausgelegt bzw. gepflanzt und mit Wasser bis zu einer Höhe von 1 cm über dem Boden angestaut. Ebenso wurde mit Reispflanzen verfahren.

Im Vorauflaufverfahren, d.h. 3-4 Tage nach dem Verpflanzen, wurden die erdindungsgemäßen Verbindungen in Form wäßriger Suspensionen oder Emulsionen ins Anstauwasser gegossen oder als Granulate ins Wasser gestreut.

Jeweils drei Wochen später wurde die herbizide Wirkung und eine eventuelle Schadwirkung gegenüber Reis optisch bonitiert. Die Ergebnisse zeigen, daß sich die erfindungsgemäßen Verbindungen zur selektiven Unkrautbekämpfung in Reis eignen.

Gegenüber bisherigen Reisherbiziden zeichnen sich die erfindungsgemäßen Verbindungen dadurch aus, daß sie zahlreiche, insbesondere auch schwer bekämpfbare Unkräuter, die aus Dauerorganen keimen, wirkungsvoll bekämpfen und dabei von Reis toleriert werden.

**Patentansprüche**

**1.** Verbindungen der Formel I oder I',

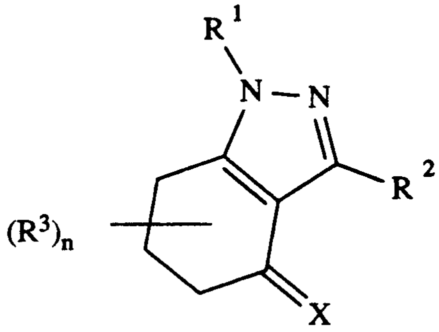

(I)

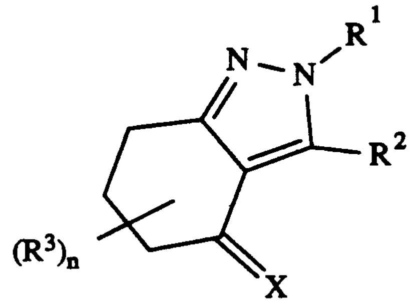

(I')

worin

X Sauerstoff- oder Schwefelatom,

$R^1$ H, $C_1$-$C_6$-Alkyl, $C_2$-$C_6$-Alkenyl, $C_2$-$C_6$-Alkinyl, $C_1$-$C_6$-Haloalkyl, ($C_1$-$C_4$-Alkoxy)-$C_1$-$C_4$-alkyl, ($C_1$-$C_6$-Alkoxy)-carbonyl, Aryloxycarbonyl, ($C_1$-$C_6$-Alkyl)-aminocarbonyl, Arylaminocarbonyl, Phenyl, substituiertes Phenyl oder Acyl,

$R^2$ Phenyl oder substituiertes Phenyl,

$R^3$ unabhängig von anderen Resten $R^3$ H oder $C_1$-$C_5$-Alkyl und

n eine ganze Zahl von 0 bis 6

bedeuten.

**2.** Verbindungen nach Anspruch 1, dadurch gekennzeichnet, daß $R^1$ H, $C_1$-$C_4$-Alkyl, $C_2$-$C_4$-Alkenyl, $C_2$-$C_4$-Alkinyl, $C_1$-$C_4$-Haloalkyl, ($C_1$-$C_2$-Alkoxy)-$C_1$-$C_4$-alkyl, ($C_1$-$C_4$-Alkoxy)-carbonyl, ($C_1$-$C_4$-Alkyl)-aminocarbonyl, Phenyloxycarbonyl, Phenylaminocarbonyl, Phenyl, wobei die 3 letztgenannten Gruppen jeweils im Phenylring unsubstituiert oder durch ein oder mehrere Reste aus der Gruppppe Halogen, $C_1$-$C_4$-Alkyl, $C_1$-$C_4$-Haloalkyl, $C_1$-$C_4$-Alkoxy, $C_1$-$C_4$-Haloalkoxy, Nitro, Carboxy und Säurederivate von Carboxy substituiert sind, oder Acyl bedeutet.

**3.** Verbindungen nach Anspruch 1 oder 2, dadurch gekennzeichnet, daß $R^2$ Phenyl, das unsubstituiert oder durch ein bis drei Reste aus der Gruppe Halogen, Nitro, $C_1$-$C_4$-Alkyl, $C_1$-$C_4$-Halogenalkyl und $C_1$-$C_4$-Alkylsulfonyl substituiert ist, bedeutet.

**4.** Verbindungen nach einem oder mehreren der Ansprüche 1 bis 3, dadurch gekennzeichnet, daß die Reste $R^3$ unabhängig voneinander vorzugsweise H oder Methyl und n eine ganze Zahl von 0 bis 4 bedeuten.

**5.** Verbindungen nach einem oder mehreren der Ansprüche 1 bis 4, dadurch gekennzeichet, daß sie Verbindungen der oben definierten Formel I sind.

**6.** Verfahren zur Herstellung von Verbindungen der Formeln I oder I' nach Anspruch 1, dadurch gekennzeichnet, daß man

a) Verbindungen der Formel II,

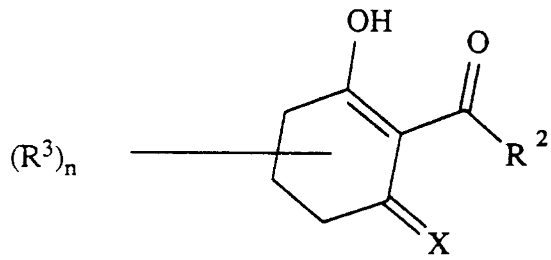

(II)

worin $R^2$ und $R^3$ und X wie bei der obengenannten Formel I bzw. I' definiert sind, mit Hydrazinderivaten der Formel III,

$R^1 - NH - NH_2$ (III)

worin $R^1$ wie bei der obengenannten Formel I bzw. I' definiert ist, umsetzt oder

b) für den Fall, daß $R^1$ = Alkyl ist, Verbindungen der genannten Formel I bzw I', in denen $R^1$ ein Wasserstoffatom bedeutet, mit geeigneten Alkylierungsmitteln umsetzt oder

c) ausgenommen zur Herstellung von Verbindungen der Formel I', Cyclohexan-1,3-dione der Formel IV mit Chlorhydrazonen der Formel V,

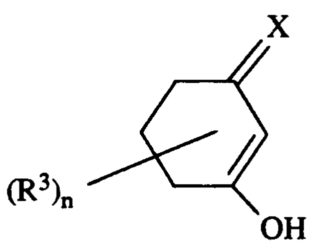

(IV)

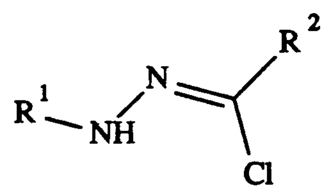

(V)

wobei $R^1$ bis $R^3$ und X wie bei der genannten Formel I bzw. I' definiert sind, in Gegenwart einer Base zu Verbindungen der genannten Formel I umsetzt.

**7.** Herbizide Mittel, dadurch gekennzeichnet, daß sie eine Verbindung der Formel I oder I' nach einem oder mehreren der Ansprüche 1 bis 5 und übliche Formulierungshilfsmittel enthalten.

**8.** Verwendung von Verbindungen der Formel I oder I' nach einem oder mehreren der Ansprüche 1 bis 5 als Herbizide.

**9.** Verfahren zur selektiven Bekämpfung von Schadpflanzen, dadurch gekennzeichnet, daß man eine wirksame Menge einer nach einem oder mehreren der Ansprüche 1 bis 5 definierten Verbindungen auf die Planzen, Planzensamen oder die Anbaufläche appliziert.

**Patentansprüche für folgenden Vertragsstaat : ES**

**1.** Verfahren zur Herstellung von Verbindungen der Formel I oder I',

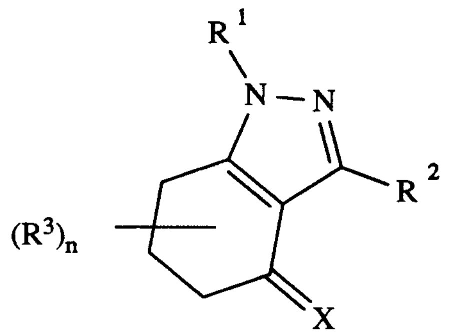

(I)

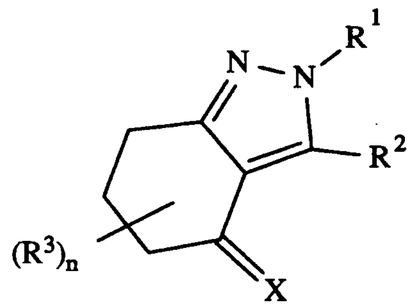

(I')

worin

X Sauerstoff- oder Schwefelatom,

$R^1$ H, $C_1$-$C_6$-Alkyl, $C_2$-$C_6$-Alkenyl, $C_2$-$C_6$-Alkinyl, $C_1$-$C_6$-Haloalkyl, ($C_1$-$C_4$-Alkoxy)-$C_1$-$C_4$-alkyl, ($C_1$-$C_6$-Alkoxy)-carbonyl, Aryloxycarbonyl, ($C_1$-$C_6$-Alkyl)-aminocarbonyl, Arylaminocarbonyl, Phenyl, substituiertes Phenyl oder Acyl,

$R^2$ Phenyl oder substituiertes Phenyl,

$R^3$ unabhängig von anderen Resten $R^3$ H oder $C_1$-$C_5$-Alkyl und

n eine ganze Zahl von 0 bis 6

bedeuten,

dadurch gekennzeichnet, daß man

a) Verbindungen der Formel II,

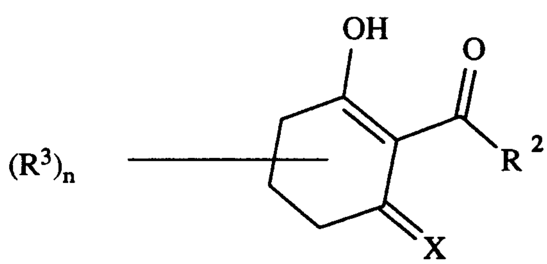

(II)

worin $R^2$ und $R^3$ und X wie bei der obengenannten Formel I bzw. I' definiert sind, mit Hydrazinderivaten der Formel III,

$R^1 - NH - NH_2$ (III)

worin $R^1$ wie bei der obengenannten Formel I bzw. I' definiert ist, umsetzt oder

b) für den Fall, daß $R^1$ = Alkyl ist, Verbindungen der genannten Formel I bzw I', in denen $R^1$ ein Wasserstoffatom bedeutet, mit geeigneten Alkylierungsmitteln umsetzt oder

c) ausgenommen zur Herstellung von Verbindungen der Formel I', Cyclohexan-1,3-dione der Formel IV mit Chlorhydrazonen der Formel V,

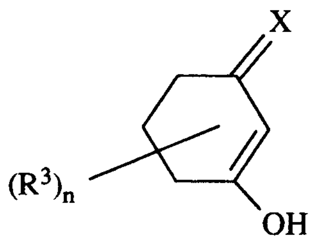

(IV)

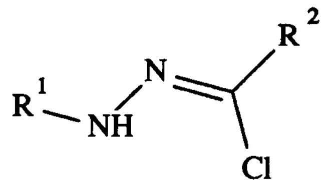

(V)

wobei $R^1$ bis $R^3$ und X wie bei der genannten Formel I bzw. I' definiert sind, in Gegenwart einer Base zu Verbindungen der genannten Formel I umsetzt.

2. Verfahren nach Anspruch 1, dadurch gekennzeichnet, daß
$R^1$ H, $C_1$-$C_4$-Alkyl, $C_2$-$C_4$-Alkenyl, $C_2$-$C_4$-Alkinyl, $C_1$-$C_4$-Haloalkyl, ($C_1$-$C_2$-Alkoxy)-$C_1$-$C_4$-alkyl, ($C_1$-$C_4$-Alkoxy)-carbonyl, ($C_1$-$C_4$-Alkyl)-aminocarbonyl, Phenyloxycarbonyl, Phenylaminocarbonyl, Phenyl, wobei die 3 letztgenannten Gruppen jeweils im Phenylring unsubstituiert oder durch ein oder mehrere Reste aus der Gruppe Halogen, $C_1$-$C_4$-Alkyl, $C_1$-$C_4$-Haloalkyl, $C_1$-$C_4$-Alkoxy, $C_1$-$C_4$-Haloalkoxy, Nitro, Carboxy und Säurederivate von Carboxy substituiert sind, oder Acyl bedeutet.

3. Verfahren nach Anspruch 1 oder 2, dadurch gekennzeichnet, daß
$R^2$ Phenyl, das unsubstituiert oder durch ein bis drei Reste aus der Gruppe Halogen, Nitro, $C_1$-$C_4$-Alkyl, $C_1$-$C_4$-Halogenalkyl und $C_1$-$C_4$-Alkylsulfonyl substituiert ist, bedeutet.

4. Verfahren nach einem oder mehreren der Ansprüche 1 bis 3, dadurch gekennzeichnet, daß die Reste $R^3$ unabhängig voneinander vorzugsweise H oder Methyl und n eine ganze Zahl von 0 bis 4 bedeuten.

5. Verfahren nach einem oder mehreren der Ansprüche 1 bis 4, dadurch gekennzeichet, daß sie Verbindungen der oben definierten Formel I sind.

6. Verfahren nach einem oder mehreren der Ansprüche 1 bis 5, dadurch gekennzeichnet, das die Umsetzung nach Variante a) in einem polaren, protischen organischen Lösungsmittel bei einer Temperaturen von Raumtemperatur bis zum Siedepunkt des Lösungsmittels durchgeführt wird.

7. Verfahren nach einem oder mehreren der Ansprüche 1 bis 5, dadurch gekennzeichnet, das die Umsetzung nach Variante c) in einem polaren, protischen oder einem aprotischen organischen Lösungsmittel durchgeführt wird.

8. Verwendung von Verbindungen der Formel I oder I', wie sie in einem oder mehreren der Ansprüche 1 bis 4 definiert sind, als Herbizide.

9. Verfahren zur selektiven Bekämpfung von Schadpflanzen, dadurch gekennzeichnet, daß man eine wirksame Menge einer nach einem oder mehreren der Ansprüche 1 bis 5 definierten Verbindungen auf die Planzen, Planzensamen oder die Anbaufläche appliziert.

Europäisches Patentamt

# EUROPÄISCHER RECHERCHENBERICHT

Nummer der Anmeldung

**EP 91 11 2818**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | SYNTHESIS November 1984, Seiten 982 - 983; P. DALLA CROCE ET AL: "A convenient synthesis of indazoles" * Seite 982, Verfahren 3; Seite 983, Verbindungen 3ba, 3bb * | 1-6 | C 07 D 231/56<br>A 01 N 43/56 |
| X | CHIMIA vol. 31, no. 2, Februar 1977, Seiten 49 - 50; W. SUCROW ET AL: "Synthesis or indazoloquinones" * Seite 49, Verbindung 1 * | 1-5 | |
| X | ZEITSCHRIFT FÜR NATURFORSCHUNG vol. 32B, no. 9-12, September 1977, Seiten 1072 - 1076; W. SUCROW ET AL: "Enhydrazine, XVIII. Einige weitere Indazol-Derivate" * Seite 1073, Verbindung 9 * | 1-5 | |
| X | JOURNAL OF THE CHEMICAL SOCIETY PERKIN TRANSACTIONS I 1973, Seiten 2697 - 2703; A.J. NUNN ET AL: "Semmler-Wolff aromatisation and abnormal Beckmann and Schmidt reactions of 3-alkyl-4-oxo1-phenyl-4,5,6,7-tetrahydroindazoles and their oximes in polyphosphoric acid" * Seite 2701, rechte Spalte, Zeilen 45, 46 * | 1-5 | |
| | | | **RECHERCHIERTE SACHGEBIETE (Int. Cl.5)** |
| X | CHEMISCHE BERICHTE vol. 109, no. 5, 1976, Seiten 1898 - 1910; V. BARDAKOS ET AL: "Enhydrazine, 14. Lactame aus 4,5,6,7-Tetrahydro-1H-4-indazolonen" * Seite 451, Verbindungen 6f, 7f; Seite 452, Verbindung 13 * | 1-5 | C 07 D 231/00<br>A 01 N 43/00 |
| X | JOURNAL OF THE CHEMICAL SOCIETY PERKIN TRANSACTIONS I 1981, Seiten 642 - 645; F.S. AL-SALEH ET AL: "Synthesis of 4-acyl- and 4-alkoxy-carbonylpyrazoles" * Seite 642, Verbindungen 2i, 2j, 2o, 2p * | 1-4 | |
| X | CHEMISCHE BERICHTE vol. 101, no. 11, 1968, Seiten 3918 - 3940; H.-J. TEUBER: "Benzolazoketone und Tetrahydroindazole aus Phenylhydrazonen cyclischer beta-Dicarbonylverbindungen" * Seite 2936, Verbindungen 20a-b * | 1-4 | |

-/-

Der vorliegende Recherchenbericht wurde für alle Patentansprüche erstellt

| Recherchenort | Abschlußdatum der Recherche | Prüfer |
|---|---|---|
| Berlin | 12 November 91 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

**EP 91 11 2818**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 059 434 (A.D. WOLF)<br>* Ansprüche 1, 32, 63, 94, 100, 103 *<br>– – – | 1,7,8 | |
| A | EP-A-0 105 721 (SUMITOMO CHEMICAL CO LTD)<br>* Ansprüche 1, 8, 9 *<br>– – – | 1,7,8 | |
| A | EP-A-0 138 527 (NIPPON KAYAKU KK)<br>* Ansprüche 1, 14, 15 *<br>– – – – – | 1,7,8 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| Berlin | 12 November 91 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument